# EUROPEAN PATENT SPECIFICATION

(11) **EP 0 588 975 B1**
(45) Date of publication and mention of the grant of the patent: **10.09.1997**
(21) Application number: 92914894.8
(22) Date of filing: 09.06.1992
(51) Int. Cl.: C08G 63/64, A61L 27/00, A61L 31/00

(54) **COPOLYMERS BASED ON CYCLIC ESTERS AND THEIR USE AS SURGICAL IMPLANTS**
COPOLYMERE AUF BASIS VON CYCLISCHEN ESTERN UND IHRE VERWENDUNG ALS CHIRURGISCHE IMPLANTATE
COPOLYMERES A BASE D'ESTERS CYCLIQUES, ET LEUR UTILISATION EN TANT QU'IMPLANTS CHIRURGICAUX

(30) Priority: 11.06.1991 NL 9101002
(43) Date of publication of application: 30.03.1994
(73) Proprietor: Rijksuniversiteit te Groningen, 9712 CP Groningen (NL)
(72) Inventor: GRIJPMA, Dirk, Wybe, NL-9722 BT Groningen (NL); VAN WIJK, Peter, Geert, Theodoor, NL-9747 AG Groningen (NL); NIJENHUIS, Atze, Jan, NL-9712 NC Groningen (NL); PENNINGS, Albertus, Johannes, NL-9331 BE Norg (NL)
(74) Representative: Smulders, Theodorus A.H.J., Ir.
(86) International application number: NL9200098
(87) International publication number: WO9222599

(56) References cited:
- EP-A- 0 427 185
- EP-A- 0 460 439
- WO-A-90/05157
- GB-A- 2 033 411
- US-A- 4 300 565

## Description

The present invention relates to biodegradable copolymers derived from cyclic esters and optionally cyclic ethers and cyclic anhydrides, whereby between 0.01 and less than 1.4 mol.% of the total cyclic ester-derived units of the coplymer are derived from trimethylene carbonate (TMC) and between 99.99 and 95 mol.% of total cyclic ester-derived units are derived from other glycolide-free cyclic esters.

### BACKGROUND OF THE INVENTION

Copolymers based on cyclic esters are known from EP-A-0.108.635 or its US counterpart US-A-4.550.449. EP-A-0.108.635 describes copolymers which can be produced from, for examples, lactides and glycolides.

Such copolymers are used in several fields. A number of polymers and copolymers of cyclic esters are biodegradable, which can present great advantages in surgically implanting objects made from such polymers or copolymers in the body of a human or animal. Because the material is biodegradable, an extra operation for the removal of the material is unnecessary and natural material can gradually take over the function of the implanted material. Such applications are generally known and are described in, for example, EP-A-0.108.635.

A drawback of the copolymers described in EP-A-0.108.635 is that the impact resistance of the materials is inadequate for certain applications. A good impact resistance is a required property of materials that must undergo sudden large impacts having great stress concentrations. This applies, for example, to materials that are used for screws in surgical implants.

A possible solution for the poor impact resistance is to reinforce the material with fibers. Such a solution is described in, for example, EP-A-334.046. A drawback of fiber-reinforced materials is that the material is less homogeneous and therefore the decomposition proceeds in unpredictable ways. Most fibers are not biodegredable while the fibers that are biodegradable are not strong enough to sufficiently reinforce a material. Moreover, combining a matrix material with fibers is an additional, complex process step.

EP-A-427.185 discloses copolymers of trimethylene carbonate and optically inactive lactides, wherein the amount of trimethylene carbonate is at least 1 wt.% (1.4 mol.%).

The aim of the present invention is to supply biodegradable copolymers of cyclic esters that possess a significantly greater impact resistance than has been previously obtained, and that do not have the disadvantage of the fiber reinforced materials.

### SUMMARY OF THE INVENTION

The present invention relates to a biodegradable copolymer as defined in claim 1.

The present invention additionally relates to such a biodegradable copolymer derived from cyclic esters which, in addition to having between 0.01 and less than 1.4 mol.% of total units derived from TMC, also has between 99.99 and 95 mol.% of the units derived from cyclic esters that are units derived from glycolide-free lactones.

The present invention further relates to a biodegradable copolymer comprised of cyclic esters which further comprises units of the copolymer which are derived from cyclic ethers or cyclic anhydrides.

The present invention additionally relates to a process for making a biodegradable copolymer derived from cyclic esters and optionally cyclic ethers and cyclic anhydrides wherein TMC monomers and non-TMC glycolide-free cyclic ester monomers are reacted together at a temperature and time sufficient to polymerize the TMC and non-TMC glycolide-free cyclic ester monomers in the presence of a catalyst, and wherein the TMC monomers are from 0.01 to less than 1.4 mol.% and the non-TMC glycolide-free cyclic esters are from 99.99 to 95 mol% of total moles of reactants and the monomers and the catalyst have a monomer/catalyst ratio of between 1000 and 30,000.

The present invention further relates to a surgically implantable, shaped article formed from a biodegradable copolymer according to claim 1.

### DETAILED DESCRIPTION OF THE INVENTION

Biodegradable copolymers of cyclic esters possessing a significantly greater impact resistance than previously obtained are achieved according to the present invention with a copolymer consisting of between 0.01 and less than 1.4 mol.% of the total units of cyclic esters and optionally cyclic ethers and cyclic anhydrides being derived from TMC and between 99.99 and 95 mol.% of the total units of cyclic esters being derived from non-TMC glycolide-free cyclic esters.

In a preferred embodiment, between 0.1 and less than 1.4 mol.% of the total units derived from cyclic ester in the copolymer are derived from TMC. In a more preferred embodiment, between 0.25 and less than 1.4 mol.% of the total units derived from cyclic esters are derived from TMC.

Surprisingly, such a biodegradable copolymer has an impact resistance that greatly exceeds the impact resistance of other copolymers derived from cyclic esters. Furthermore, such a copolymer also has a larger tensile strength than other copolymers derived from cyclic esters.

Examples of cyclic esters are lactones and cyclic carbonates. Examples of lactones are lactide, ε-caprolactone, dioxanone, 1,4-dioxane-2,3-dione, beta-propiolactone, beta-butyrolactone, gamma-butyrolactone or pivalolactone.

Examples of cyclic carbonates are the already mentioned TMC, 2,2-dimethyl-trimethylene carbonate.

In a preferred embodiment, the other cyclic monomer is a lactide such as L-lactide, D-lactide or D,L-lactide or combinations thereof. In a more preferred embodiment L-lactide is used.

Glycolides are not used in the invention because glycolides result in a copolymer composition which hydrolyzes more quickly than a similar composition which contains no glycolides. When such a copolymer composition derived from glycolides is formed into an article which is surgically implanted into the body, the risk of a tissue reaction is greater because of the faster hydrolysis. Such a tissue reaction is significantly less likely to happen with a copolymer composition derived from lactides rather than glycolides.

Cyclic esters generally have a schematic structural formula according to Figure (I):

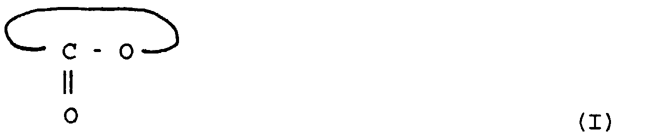

Additionally, cyclic esters are also understood to include cyclic diesters with a schematic structural formula according to Figure (II) :

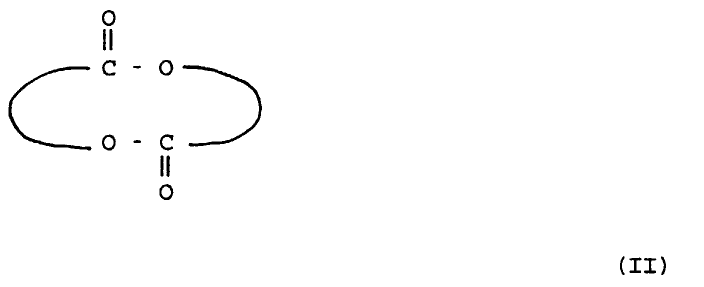

Lactide is generally understood to be a dilactide with a structural formula according to Figure (III):

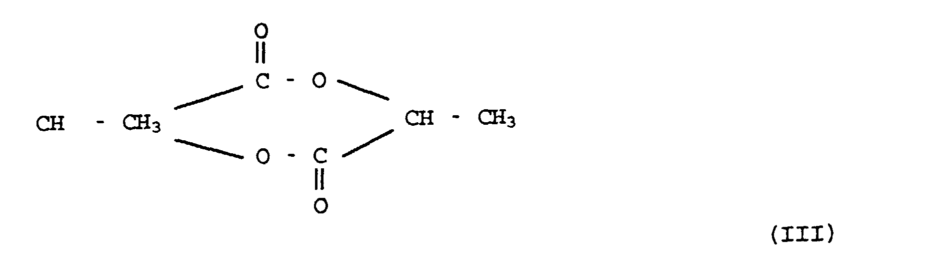

A lactide is usually formed as a result of ring formation by esterification of two lactic acids.

TMC is a cyclic carbonate. A carbonate can be regarded as a diester. A cyclic carbonate has a schematic structural formula according to Figure (IV):

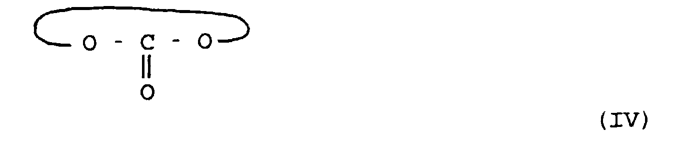

TMC has a structure according to Figure (V):

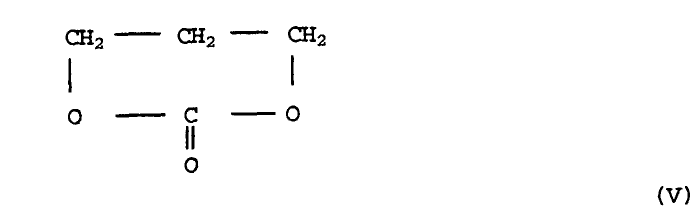

Optionally, use can be made of substituted TMC such as 2,2-dimethyl TMC. TMC is also known as 1,3-dioxan-2-one.

It is also possible to incorporate all conceivable other glycolide-free monomers in the copolymer. Preferably, monomers are incorporated that can react by ring-opening polymerization. These monomers can be chosen from, for example, the group consisting of cyclic ethers and cyclic anhydrides.

In addition, the following monomers can be copolymerized:
alpha-hydroxybutyric acid,
alpha-hydroxyisobutyric acid,
alpha-hydroxyvaleric acid,
alpha-hydroxyisovaleric acid,
alpha-hydroxycaproic acid,
alpha-hydroxyisocaproic acid,
alpha-hydroxy-alpha-ethylbutyric acid,
alpha-hydroxy-beta-methylvaleric acid,
alpha-hydroxyheptanoic acid,
alpha-hydroxyoctanoic acid,
alpha-hydroxydecanoic acid,
alpha-hydroxymyristinic acid,
alpha-hydroxystearic acid or combinations thereof or the intermolecular cyclic esters of these monomers. A further list is given in GB-A-1.604.177 or divisional patent GB-A-604.178 or later filed counterpart GB-A-2.033.411 or their American counterparts US-A-4.300.565 and US-A-4.243.775.

Copolymers of cyclic esters including TMC are described in GB-A-2.033.411. The disclosure of this patent describes copolymers that are obtained through copolymerization of glycolide and TMC units. This patent teaches that it is possible to use between 1 and 99% TMC, preferably between 1 and 50%, more preferably between 1 and 35% and most preferably between 10 and 20%. The examples show a TMC percentage of 8% and higher. Thus, this patent teaches TMC percentages that are higher than those according to the present invention. This patent does not describe any advantages of using a TMC percentage according to the present invention.

The biodegradable copolymer can be synthesized by the methods known to a person skilled in the art that are described in general terms in, for example, EP-A-0.108.365. For example, TMC and the other monomer(s) can be reacted together in the melt at a polymerization temperature of 110°C, for 240 hours, using tin octoate as the catalyst.

The preferred polymerization temperature used in the reaction depends on, for example, the manner of polymerization and the concentration of the catalyst. In general, a polymerization temperature is chosen that is in a range from the melting points of the cyclic ester monomers (either TMC or the other monomers) up to and including 200°C.

The polymerization can be conducted for as short a time as few minutes or for as long a time as a few weeks. The range of polymerization time is usually between 30 minutes and 2 weeks. A preferred range is between 20 and 200 hours. A more preferred range for the polymerization time is between 60 and 150 hours. The required or preferred polymerization time depends on the temperature and catalyst concentration chosen.

The polymerization reaction can take place in the form of a melt, a solution, an emulsion, a suspension or in whatever other manner than can be used. Preferably, the reaction takes place in the form of a melt.

Catalysts which can be used for the polymerization reaction are, for example, tin octoate, anitmony trifluoride, metallic zinc (powder), dibutyltin oxide and tin oxalate. Other catalysts are described in EP-A-0.098.394 or its US counterpart US-A-4.429.080. In addition, virtually all reesterification catalysts which would be known to a person skilled in the art can be used.

The monomer/catalyst molecular ratio (mon/cat ratio) is preferably between 1,000 and 300,000, more preferably between 5,000 and 30,000.

The reaction can take place in a vacuum, for example in a sealed ampule, or in an inert atmosphere such as nitrogen.

The biodegradable copolymer obtained from the polymerization reaction can subsequently be purified or it can be used directly after polymerization. The material that is obtained after polymerization, i.e. without for example an additional purification or remelting step, is known as "as polymerized" material. Preferably, the material is used "as polymerized".

Objects can be made from the biodegradable copolymer by, for example, remelting, injection molding, dissolution and recrystallization, stretching, milling, turning and optionally any other type of processing which is available. The molecular structure of the "as polymerized" material is virtually unaffected by milling or turning, for example.

The biodegradable copolymers according to the present invention can be used in surgical procedures, as, for example, in surgical implants. It is possible to manufacture objects with an impact resistance of at least 11 kJ/m² and as high as 100 kJ/m² and a tensile strength of at least 51 MPa and as high as 200 MPa. Objects of similar composition having such superior properties have never been described in the literature.

In a preferred embodiment, objects according to the present invention are used in surgical implants in wich the objects become subjected to great mechanical loads. The screws that are used to fasten bone plates to bone are typical embodiments. Other embodiments can include pins or other surgical fasteners.

The present invention will be elucidated with the aid of the following examples.

The impact resistance, I.R., was determined using unnotched Dynstat specimens according to DIN 53453.

The intrinsic viscosity, [η], was measured with the aid of an Ubbelohde Viscosimeter, type Oa, according to ASTM D-445, at 25°C, in chloroform.

The melting temperature, Tₘ, the entropy, ΔH, and the glass transition temperature, T_{g}, were determined by a DSC measurement of specimens of approx. 10 mg using a calibrated Perkin Elmer DSC-7, at a scanning rate of 10°C min⁻1. The T_{g} was determined in a second measuring step after cooling from the melt.

The tensile strenght, σ, and the elongation at break, ε, were determined with the aid of a stress/strain curve, which was obtained using an Instron 4301 tensile tester (Limited High Wycombe), with a 5000-N load cell and a crosshead speed of 10 mm min⁻1.

### Example I

### Polymerization of an L-lactide-TMC copolymer

58.9 g of L-lactide (obtained from C.C.A. Biochem, the Netherlands) and 0.6 g of TMC (obtained by depolymerization of low-molecular weight poly-TMC produced by polycondensation of diethyl carbonate and propanediol) were melted in a glass ampule with a lactide:TMC molar ratio of 98.6:1.4. 11 mg of tin octoate was added (monomer/catalyst ratio of 15,000:1). The melt was kept at 100°C for 10 days. The amount of TMC incorporated was determined to the nearest 1.0 mol.% by 300-MHz NMR. The impact resistance was determined in the "as polymerized" copolymer thus obtained and in an amount of remelted (compression molded) material. In addition, the tensile strength and the elongation at break were measured. The results are shown in Table 1.

### Examples II through XI

### Different percentages of TMC

The procedure of example I was repeated using different percentages of TMC. The percentages and the results are shown in Table 1.

### Comparative experiments A1-A9

The procedure of example I was repeated using higher percentages of TMC. The percentages and the results of the measurements are shown in Table 1.

### Comparative experiment A10

The procedure of example I was repeated without TMC. The results of the measurements are shown in Table 1.

**TABLE 1**

| Results of the polymerization and the measurements | | | | | | | | |
|---|---|---|---|---|---|---|---|---|
| Example | TMC* (mol.%) | η (dl/g) | Tm (°C) | ΔH (j/g) | Tg (°C) | σ (MPa) | ε (%) | I.R. (kj/m²) |
| II | 0.41 | - | 192.4 | 72.0 | 56.9 | 52.0 | 5.9 | 17.7 |
| III | 0.5 | 9.0 | - | - | - | 53.6 | 7.6 | 23.7 |
| IV | 0.7 | - | 185.3 | 64.9 | 56.1 | 58.0 | 9.3 | 28.9 |
| I | 1.0 | 10.2 | - | - | - | 53.2 | 8.1 | 34.0 |
| V | 1.1 | 10.2 | 190.8 | 65.0 | 56.7 | 57.5 | 14.0 | 24.9 |
| VI | 1.1 | 8.0 | - | - | - | 55.4 | 12.1 | - |
| VII | 1.2 | - | - | - | - | 54.9 | 8.6 | 16.2 |
| VIII** | 1.4 | - | 186.8 | 67.5 | 51.4 | 57.0 | 19.9 | 22.0 |
| IX** | 1.46 | - | 190.5 | 70.8 | 55.3 | 55.6 | 17.4 | 14.6 |
| X** | 1.5 | 8.3 | - | - | - | - | - | 18.0 |
| XI** | 2.8 | - | 188.3 | 69.2 | 55.8 | - | - | 11.2 |
| | | | | | | | | |

| Comp.Exp. | | | | | | | | |
|---|---|---|---|---|---|---|---|---|
| A1 | 5.6 | 7.2 | 185.8 | 66.2 | 54.1 | - | - | 6.0 |
| A2 | 73 | - | 185.6 | 60.0 | - | 50.9 | 13.3 | 5.8 |
| A3 | 8.4 | - | 179.4 | 50.8 | 52.9 | 50.4 | 15.6 | 6.8 |
| A4 | 9.7 | - | 168.8 | 64.5 | - | - | - | 5.6 |
| A5 | 10.6 | 5.4 | 185.0 | 50.9 | - | 48.7 | 15.1 | - |
| A6 | 10.8 | 2.7 | - | - | - | - | - | 5.5 |
| A7 | 15.5 | 7.8 | 183.5 | 56.0 | 47.9 | 34.8 | 11.9 | 5.0 |
| A8 | 19.1 | - | 179.7 | 51.5 | 47.4 | - | - | 6.2 |
| A9 | 31.1 | - | 173.8 | 38.7 | 37.3 | 27.6 | 33.0 | 29.7 |
| A10 | 0 | 9.6 | 192.1 | 76.0 | 60.0 | 59.5 | 12.2 | 11 |

| | | | | | | | | |
|---|---|---|---|---|---|---|---|---|
| * incorporated into the polymer; determined by means of NMR. | | | | | | | | |
| ** comparative Example | | | | | | | | |

The impact resistance of the material appears to be fairly high at a higher mol.% of TMC. Surprisingly, the material also appears to show a great increase in the impact resistance at low TMC concentrations. This peak in the impact resistance lies around 1 mol.% TMC and amounts to 34 kJ/m² ·

The copolymers containing between 1.4 and 20 mol.% TMC had a considerably poorer impact resistance.

At the TMC concentration according to the present invention, the tensile strength of the copolymer is virtually as high as that of an "as polymerized" homo-L-lactide, as determined in comparative experiment A10, whereas the tensile strength of the copolymers with higher TMC percentages is substantially poorer.

The impact resistance values measured for the remelted and compression molded samples are much lower.

## Claims

1. Biodegradable copolymer consisting of units derived from glycolide-free cyclic esters and optionally cyclic ethers and cyclic anhydrides, wherein between 0.01 and less than 1.4 mol.% of the total units derived from cyclic esters are units derived from trimethylene carbonate.

2. Copolymer according to claim 1, werein between 99.99 and 95 mol.% of the units derived from cyclic esters are derived from glycolide-free lactones.

3. Copolymer according to claim 2, wherein the glycolide-free lactones are lactides.

4. Copolymer according to claim 3, wherein the lactides are selected from the group consisting of D-lactides, L-lactides, D,L-lactides and mixtures thereof.

5. Copolymer according to claim 4, wherein the lactides are L-lactides.

6. Copolymer according to any one of claims 1-5, wherein between 0.1 and less than 1.4 mol.% of the total units derived from cyclic esters are derived from TMC.

7. Copolymer according to claim 6, wherein between 0.25 and less then 1.4 mol.% of the total units derived from cyclic esters are derived from TMC.

8. Copolymer according to any one of claims 1-7, further comprising units derived from cyclic ethers or cyclic anhydrides.

9. Process for the production of a biodegradable copolymer according to any one of the claims 1-8, comprising reacting glycolide-free cyclic esters and trimethylene carbonate and optionally cyclic ethers and cyclic anhydrides in the presence of a catalyst, wherein between 0.01 and less than 1.4 mol.% of the total units derived from cyclic esters are units derived from trimethylene carbonate and the other cyclic ester monomers are from 99.99 to 95 mol.% of the total mols of reactants and the monomers and the catalyst have a monomer/catalyst molecular ratio of between 1000 and 30,000.

10. The process according to claim 9, wherein the reacting step is conducted at a temperature between a melting point of the monomers and 200°C for a period between 30 minutes and 2 weeks.

11. A surgically implantable, shaped article formed from a biodegradable copolymer according to any one of claims 1-8 or obtained with the aid of a process according to claim 9.

12. A surgically implantable, shaped article according to claim 11, wherein the article has an impact resistance of from 11 kJ/m² to 100 kJ/m² and a tensile strength of from 51 to MPa to 200 MPa.

13. A surgically implantable, shaped article according to claim 11 or claim 12, wherein the article is a screw, pin or other surgical fastener.

## Patentansprüche

1. Biologisch abbaubares Copolymer, bestehend aus Einheiten, die sich von Glykolid-freien Estern und gewünschtenfalls cyclischen Ethern und cyclischen Anhydriden ableiten, bei dem zwischen 0,01 und weniger als 1,4 mol.% der gesamten von cyclischen Estern abgeleiteten Einheiten sich von Trimethylencarbonat ableiten.

2. Copolymer gemäß Anspruch 1, bei dem zwischen 99,99 und 95 mol.% der von cyclischen Estern abgeleiteten Einheiten von Glykolid-freien Lactonen abgeleitet sind.

3. Copolymer gemäß Anspruch 2, bei dem die Glykolid-freien Lactone Lactide sind.

4. Copolymer gemäß Anspruch 3, bei dem die Lactide ausgewählt sind aus der Gruppe, bestehend aus D-Lactiden, L-Lactiden, D,L-Lactiden und Mischungen davon.

5. Copolymer gemäß Anspruch 4, bei dem die Lactide L-Lactide sind.

6. Copolymer gemäß einem der Ansprüche 1 bis 5, bei dem zwischen 0,1 und weniger als 1,4 mol.% der gesamten von cyclischen Estern abgeleiteten Einheiten sich von TMC ableiten.

7. Copolymer gemäß Anspruch 6, bei dem zwischen 0,25 und weniger als 1,4 mol.% der gesamten von cyclischen Estern abgeleiteten Einheiten sich von TMC ableiten.

8. Copolymer gemäß einem der Ansprüche 1 bis 7, umfassend weiterhin Einheiten, die sich von cyclischen Ethern oder cyclischen Anhydriden ableiten.

9. Verfahren zur Herstellung eines bioabbaubaren Copolymers gemäß einem der Ansprüche 1 bis 8, umfassend das Umsetzen von Glykolid-freien cyclischen Estern und Trimethylencarbonat, und gewünschtenfalls cyclischen Ethern und cyclischen Anhydriden in Gegenwart eines Katalysators, bei dem zwischen 0,01 und weniger als 1,4 mol.% der gesamten von cyclischen Estern abgeleiteten Einheiten Einheiten sind, die sich von Trimethylencarbonat ableiten, und die anderen cyclischen Estermonomeren 99,99 bis 95 mol.% der gesamten Mole der Reaktanten sind, und die Monomeren und der Katalysator ein Monomer/Katalysator-Molekularverhältnis zwischen 1000 und 30.000 haben.

10. Verfahren gemäß Anspruch 9, bei dem die Umsetzungsstufe bei einer Temperatur zwischen dem Schmelzpunkt der Monomeren und 200°C während eines Zeitraums zwischen 30 Minuten und 2 Wochen erfolgt.

11. Ein chirurgisch implantierbarer, geformter Gegenstand, gebildet aus einem bioabbaubaren Copolymer gemäß einem der Ansprüche 1 bis 8 oder erhalten mit Hilfe eines Verfahrens gemäß Anspruch 9.

12. Ein chirurgisch implantierbarer, geformter Gegenstand gemäß Anspruch 11, bei dem der Gegenstand eine Schlagfestigkeit von 11 kJ/m² bis 100 kJ/m² und eine Zugfestigkeit von 51 MPa bis 200 MPa hat.

13. Ein chirurgisch implantierbarer, geformter Gegenstand gemäß Anspruch 11 oder Anspruch 12, bei dem der Gegenstand eine Schraube, ein Nagel oder anderes chirurgisches Befestigungsmittel ist.

## Revendications

1. Copolymère biodégradable consistant en unités dérivées d'esters cycliques exempts de glycolides et éventuellement d'éthers cycliques et d'anhydrides cycliques, dans lequel entre 0,01 et moins de 1,4 mol % des unités totales dérivées d'esters cycliques sont des unités dérivées de carbonate de triméthylène.

2. Copolymère selon la revendication 1, dans lequel entre 99,99 et 95 mol % des unités dérivées d'esters cycliques sont dérivées de lactones exemptes de glycolides.

3. Copolymère selon la revendication 2, dans lequel les lactones exemptes de glycolides sont des lactides.

4. Copolymère selon la revendication 3, dans lequel les lactides sont choisis dans le groupe consistant en les D-lactides, les L-lactides, les D,L-lactides et leurs mélanges.

5. Copolymère selon la revendication 4, dans lequel les lactides sont des L-lactides.

6. Copolymère selon l'une quelconque des revendications 1 à 5, dans lequel entre 0,1 et moins de 1,4 mol % des unités totales dérivées d'esters cycliques sont dérivées de TMC.

7. Copolymère selon la revendication 6, dans lequel entre 0,25 et moins de 1,4 mol % des unités totales dérivées d'esters cycliques sont dérivées de TMC.

8. Copolymère selon l'une quelconque des revendications 1 à 7, comprenant en outre des unités dérivées d'éthers cycliques ou d'anhydrides cycliques.

9. Procédé de production d'un copolymère biodégradable selon l'une quelconque des revendications 1 à 8, comprenant la réaction d'esters cycliques exempts de glycolides et de carbonate de triméthylène et éventuellement d'éthers cycliques et d'anhydrides cycliques en présence d'un catalyseur, dans lequel entre 0,01 et moins de 1,4 mol % des unités totales dérivées d'esters cycliques sont des unités dérivées de carbonate de triméthylène et les autres monomères esters cycliques représentent 99,99 à 95 mol % des moles totales des corps réagissants et les monomères et le catalyseur ont un rapport moléculaire monomère/catalyseur compris entre 1 000 et 30 000.

10. Procédé selon la revendication 9, dans lequel l'étape de réaction est conduite à une température comprise entre le point de fusion des monomères et 200°C pendant une durée comprise entre 30 min et 2 semaines.

11. Article façonné implantable par voie chirurgicale formé à partir d'un copolymère biodégradable selon l'une quelconque des revendications 1 à 8 ou obtenu au moyen d'un procédé selon la revendication 9.

12. Article façonné implantable par voie chirurgicale selon la revendication 11, dans lequel l'article a une résistance au choc de 11 kJ/m² à 100 kJ/m² et une résistance à la traction de 51 MPa à 200 MPa.

13. Article façonné implantable par voie chirurgicale selon la revendication 11 ou la revendication 12, dans lequel l'article est une vis, une broche ou une autre attache chirurgicale.
